Europäisches Patentamt

European Patent Office

Office européen des brevets

(19)

(11) Publication number: **0 166 130**
**A2**

(12)

# EUROPEAN PATENT APPLICATION

(21) Application number: **85105514.5**

(51) Int. Cl.⁴: **C 12 N 5/00**

(22) Date of filing: **06.05.85**

---

(30) Priority: **11.05.84 JP 94270/84**

(43) Date of publication of application: **02.01.86**
**Bulletin 86/1**

(84) Designated Contracting States: **CH DE FR GB IT LI NL SE**

(71) Applicant: **OTSUKA PHARMACEUTICAL CO., LTD., 9, Kandatsukasacho 2-chome, Chiyoda-ku Tokyo 101 (JP)**

(72) Inventor: **Mizuta, Toshinobu, 1671-50, Yamanamachi, Takasaki-shi Gunma-ken (JP)**
Inventor: **Adachi, Masakazu, 3493-9, Ishihara-cho, Takasaki-shi Gunma-ken (JP)**

(74) Representative: **Wächtershäuser, Günter, Dr., Tal 29, D-8000 München 2 (DE)**

---

(54) Additives for use in cell culture, culture medium for non-serum cultivation and method of cell culture using them.

(57) Additives for use in cell culture comprise ovomacroglobulin obtainable from egg-white protein. The additives are incorporated into culture mediums as a subsituent to serum, which has been normally added to conventional nutrition mediums in the field of cell culture, thus establish a non-serum cultivation.

The ovomacroglobulin desirably promotes the propagating property of cultivated cells and facilitate the isolation of aimed substances produced by the cultured cells.

## FIELD OF THE INVENTION

This invention concerns a technic for cell culture and, more specifically, additives for use in cell culture for setting satisfactory cell culture conditions.

## BACKGROUND OF THE INVENTION

With the progress of the biotechnology in recent years, cell culture technics as the basic technical means have become more and more important and, among all, various studies have been made particularly on the culture mediums suitable to the cell culture.

At present, various nutrition mediums (basal mediums) are commercially available and it is generally known that the addition of serums, particularly, bovine fetal serums (FCS) is required in the culture medium for cell culture.

However, since these serums comprise various kinds of ingredients, it is extremely difficult and even impossible depending on the case to isolate and collect useful substances that cells produce, for example, from a cell culture liquid. Furthermore, the serums have disadvantages that they are difficult to obtain and are expensive, as well as provide no reproducible effect in the cultivation.

0166130

The present inventors have made an earnest study on the cell culture conditions, particularly, on substituent ingredients for the serum and, as the result, have accomplished this invention based on the findings that ovomacroglobulin obtainable from egg-white has an excellent cell propagation effect and has no disadvantages as in the serum.

### SUMMARY OF THE INVENTION

Accordingly, an object of this invention is to provide additives for use in cell culture comprising ovomacroglobulin.

Another object of this invention is to provide a culture medium for use in non-serum cultivation comprising ovomacroglobulin.

A further object of this invention is to provide a method of cell culture carried out under the non-serum conditions.

The ovomacroglobulin used in this invention is able to maintain an activity of cultivated cells and extremely desirably promotes the propagating property thereof. Further, since this is a substance composed of a single ingredient, it causes no hindrance upon isolating to collect useful substances and provides

- 3 -

reproducible effect in the cultivation. This is extremely advantageous in the cytotoxic test of chemicals by using the cultured cells.

### BRIEF DESCRIPTION OF THE DRAWINGS

Figure 1 is a graph showing the propagating effect of the adherent cells by the addition of "Additives No. 1" for use in the cell culture according to this invention,

Figure 2 is a photograph showing the propagating state of the cells when the adherent cells are cultivated in RPMI-1640 medium,

Figures 3 through 6 are photographs showing the propagating state of the cells when adherent cells are cultivated in RPMI-1640 medium with "Additives No. 2",

Figure 7 is a graph showing the propagating effect of the floating cells by adding "Additives No. 1",

Figure 8 is a graph showing the relationship between the addition amount of "Additive No. 1" and the number of floating cells,

Figures 9 and 10 are graphs showing the cell metabolic performance and the viability by using "culture medium No. 2" for use in the cell culture according to this invention in comparison with those by using the culture mediums with bovine fetal serum,

Figures 11 and 12 are graphs showing the increase in the number of cells when subcultivation is carried out by using the same mediums as above,

Figure 13 is a graph showing the accumurated number of cells when the subcultivation is carried out by using the same medium as above.

## DETAILED DESCRIPTION OF THE INVENTION AND AND PREFERRED EMBODIMENTS

The ovomacroglobulin is known as a high molecular weight glycoprotein in egg-white and the preparation method therefor has also already been known (Feeney R.E., et al.; Comp. Biochem, Physiol., 54A, 281 (1976), Ikai A. et al.; J. Biochem. 92, 1679 - 1682 (1982), and 93, 121 - 127 (1983); and Nagase H, et al.; J. B. C., vol. 258, NO. 12, 7481 - 7489 (1983), etc.). The egg-white as the starting material for preparing ovomacroglobulin has no particular limitation and those from various kinds of animals can be used. Generally, egg-white of chicken, duck, quail, turkey and the like which is easily available is preferred. Furthermore,
there are no particular limitations for the preparation
method, and various processing procedures that utilize
physicochemical properties or the like of
ovomacroglobulin, for example, treatment with protein
precipitating agent, molecular sieve chromatography (gel
filtration), ion exchange chromatography,
centrifugation, electrophoresis and dialysis can be used
alone or in combination, in the same manner as the
method usually employed for the separation of protein
ingredients.

For instance, ovomacroglobulin can be obtained
as a glycoprotein with 600,000 to 800,000 of molecular
weight by mixing egg-white in a water-soluble solvent
such as a tris-hydrochloric acid buffer solution,
removing insoluble protein ingredients such as ovomucin
and then subjecting the residue to the gel filtration.

For the additives for use in cell culture
according to this invention comprising ovomacroglobulin
as the essential ingredient, there is no particular
limitation for the state thereof and the ingredients
optionally blended therewith, unless the effect thereof
is not impaired. As the example of the preparation,
solution of aqueous solvent, freeze-dried powder or the
like may be mentioned.

As described later, satisfactory cell culture is possible by the use of a basal medium comprising ovomacroglobulin and, among all, more suitable culture conditions can be established in the case where low density lipoprotein (LDL), particularly, yolk LDL, catalase, albumin, insulin, transferrin and selenium are present together. Accordingly, considering that there may be present basal mediums lacking any of these ingredients, provision of additives comprising ovomacroglobulin and, in addition, at least one of these ingredients optionally blended therewith is convenient upon use thereof. It will be apparent that this invention includes those additives further blended with these ingredients. In the case of providing additives incorporating these ingredients, while there are no particular restrictions for the blending ratio thereof, a preferred range for each ingredient is as below:

| Ingredient | Weight ratio |
| --- | --- |
| Ovomacroglobulin | 1 - 100 |
| LDL (as the amount of triglyceride) | 0.1 - 500 |
| Catalase | $2.4 \times 10^{-6}$ -24 |
| Albumin | 10 - 10000 |
| Insulin | 1 - 100 |

0166130

| | |
|---|---|
| Transferrin | 1 - 100 |
| Selenium | 0.001 - 0.1 |

The additives according to this invention can, of course, be incorporated and blended with usual diluent, ingredients for the basal medium, known propagation sustaining ingredients, preservatives and the likes.

By the additives for use in cell culture according to this invention are used being added to usual basal mediums, cell culture can be carried out in the same conditions as those in the conventional method of using serums. The basal medium includes, for example, CEM medium, CMRL-1066 medium, DM-160 medium, Eagle's minimum essential medium (Eagle's MEM), autoclavable MEM, Fisher's medium, F-10 or F-12 medium, L-15 medium, NCTC-109 medium, RPMI-1640 medium, McCoy's 5A medium and the like. In this specification, the basal culture medium is defined to include above-mentioned commercial products, those mediums having approximately the same ingredient compositions and those mediums prepared newly to have such compositions.

The additives for use in cell culture according to this invention are added in such an amount

- 8 -

that the amount of ovomacroglobulin ranges from 0.1 µg to 2 mg, preferably 0.1 µg to 1mg, and more preferably 1 to 100 µg to 1 ml of the basal culture medium.

When the cultivation is carried out at pH from 6 to 8 and temperature of about 37°C by using the culture medium incorporated with the additives according to this invention, an extremely preferred result can be obtained.  The above-mentioned medium can be used for the culture of various kind of cells of animal origin and the cells may be either of adherent or floating cells, or established cells, fresh malignant cells or normal cells.

As another embodiment of this invention, culture medium for use in non-serum cultivation comprising an effective amount of ovomacroglobulin can be mentioned.  It is necessary that the above-mentioned culture medium contains, as an essential ingredient, ovomacroglobulin. A concentration of ovomacrogrobulin is generally of from 0.1 µg to 2 mg/ml, preferably from 0.1 µg to 1 mg/ml, more preferably, from 1 to 100 µg/ml blended therewith.  There is no particular restriction for other ingredients, and those mediums prepared by blending the above-mentioned basal culture medium further with ovomacroglobulin can be exemplified.

As a more preferred culture medium, there can be mentioned the above-exemplified basal medium so prepared as to contain:

1 - 100 µg/ml of ovomacroglobulin,

0.1 - 500 µg/ml of yolk LDL (for instance as the amount of triglyceride),

1 - 100 µg/ml of transferrin,

1 - 100 µg/ml of insulin,

1 - 100 ng/ml of selenium,

10 µg - 10 mg/ml of albumin, and

about $10^{-14} - 10^{-7}$ M of catalase (approximate concentration).

As a further embodiment of this invention, a method of cell culture under the non-serum conditions by using the above specified culture medium can be mentioned. According to this method, the cell culture can be carried out extremely desirably while satisfactorily maintaining the functions of the cells.

This invention will now be explained referring to reference examples, examples and test examples.

**Reference Example 1**

Egg-white separated from twenty chicken eggs were suspended into an equi volume of 25 mM of trishydrochloric acid buffer solution (pH 7.6) added
with 1% NaCl, and polyethylene glycol (MW = 7500,
manufactured by Wako Pure Chemical Industries) was added
so as to adjust the concentration to 2%, applying
centrifugation, (10,000 rpm x 10 min), and the
supernatant was collected.  Polyethylene glycol was
added to the portion so as to adjust the concentration
to 10% and then applied with centrifugation (10,000 rpm
x 10 min) to collect the precipitated portion.  The
precipitates were dissolved in the buffer solution and
further subjected to centrifugation (10,000 rpm x 10
min), to collect the supernatant.  The collected portion
was subjected column chromatography using a column (2
liters) of CL-6B (manufactured by Pharmacia Fine
Chemicals Co), eluted out by the same buffer solution as
above to obtain ovomacroglobulin having thermolysin
enzyme inhibitive activity.  Molecular weight: about
700,000.

**Example 1**

(1)  Ovomacroglobulin obtained in the
Reference Example 1 was subjected to dialysis in 25 mM
of tris-hydrochloric acid buffer solution with 1% NaCl,
adjusted to 0.6 mg protein amount/ml and preserved under
4°C.  This is referred to as "Additive No. 1".

The amount of protein was determined by "Tonein TP (registered trade mark)" manufactured by Otsuka Assay Lab., Otsuka Pharmaceutical Co., Ltd. (this step is common throughout the subsequent examples).

(2) Ovomacroglobulin obtained in the Reference Example 1 was subjected to dialysis in 0.01 M phosphate buffer solution (pH = 7.4) and prepared into 1 mg of protein amount/ml. This is referred to hereinafter as "Additive No. 2".

(3) Ovomacroglobulin derived from alligator egg-white supplied from Prof. Ikai, Tokyo University (J. Biochem. 93, 121 - 127 (1983)) and in the same manner as in (1) above, it was prepared into 10 mg protein amount/ml. This is referred hereinafter as "Additive No. 3".

(4) A portion of the above-mentioned additive (1) was freeze-dried. This is referred to as "Additive No. 4".

Test Example 1

The following culture tests were carried out using adherent cells QG-90 (Human lung cancer cell line: Cancer Res., 42, 601 - 608 (1982)):

(1)    Each 1 ml of QG-90 prepared to 1.0 x $10^5$ cells/ml in RPMI-1640 medium (GIBCO Co.) comprising 10 M of 4-(2-hydroxyethyl)-1-piperadine ethane sulfonic acid (HEPES) was placed in a 24 well plate (manufactured by Nunc Co.) and cultivated in a 5% carbon dioxide gas incubator under 37°C.   The additives No. 1 according to this invention were added at various concentrations, and the cell propagating performance after 18 hours and 48 hours from the start of the cultivation was measured by MTT Assay (Progress of Medicine, vol. 128, No. 1, p9 - 10 (1984)) was measured.

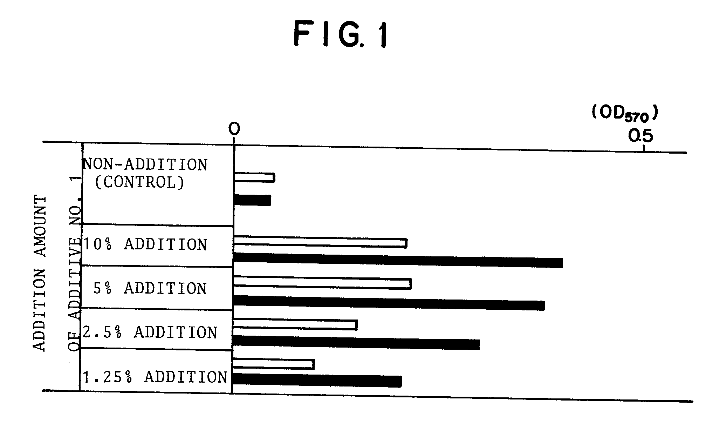

The results are shown in Figure 1.   In Figure 1, white bar represents absorbance at 570 nm ($OD_{570}$) measured 18 hours after, and black bar represents $OD_{570}$ nm measured 48 hours after respectively.

The MTT assay reflects the active mitochondria and it can be seen from Figure 1 that, by the addition of additive according to this invention, extremely desirable nutrition conditions are set.

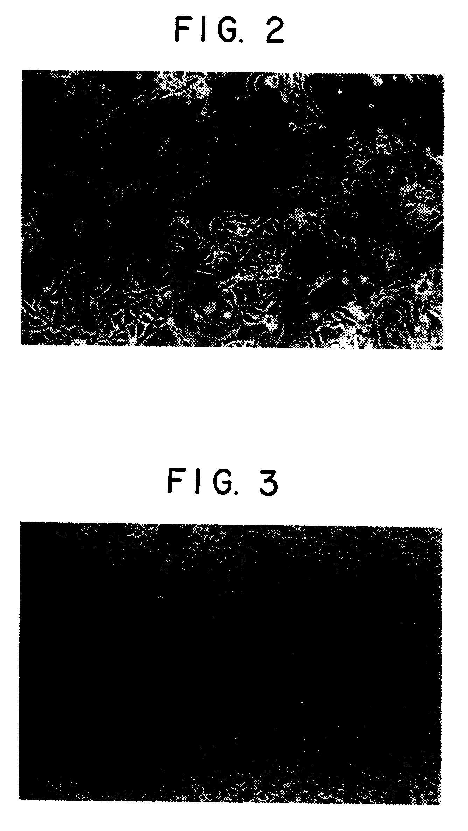

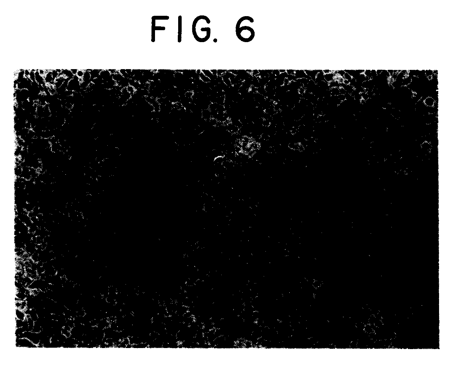

(2)    In the same manner as above (1), QG-90 was cultivated for 5 days under 37°C by using the culture medium of RPMI-1640 incorporated with the "Additive No. 2" at various concentrations.   The results are shown in Figures 2 through 6.   The figures represent respectively as:

Figure 2:   non addition (control medium)

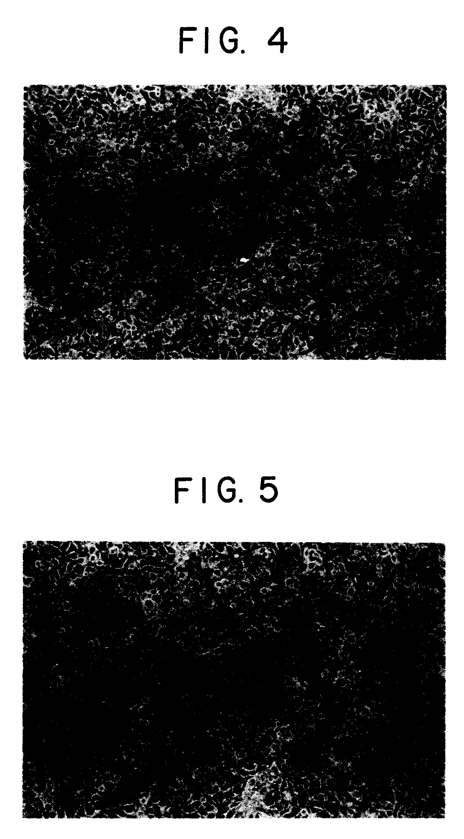

Figure 3:   0.1 mg of protein/ml added

Figure 4:   0.05            "

Figure 5:   0.025           "

Figure 6:   0.013           "

From Figures 2 through 6, it can be seen that the groups to which the additive according to this invention were added show extremely satisfactory propagation.

**Test Example 2**

The following culture tests were carried out by using floating cells Raji (Burkitt's human lymphoma cell line:  J. Nat. Cancer Inst. 34, 231 (1965)).

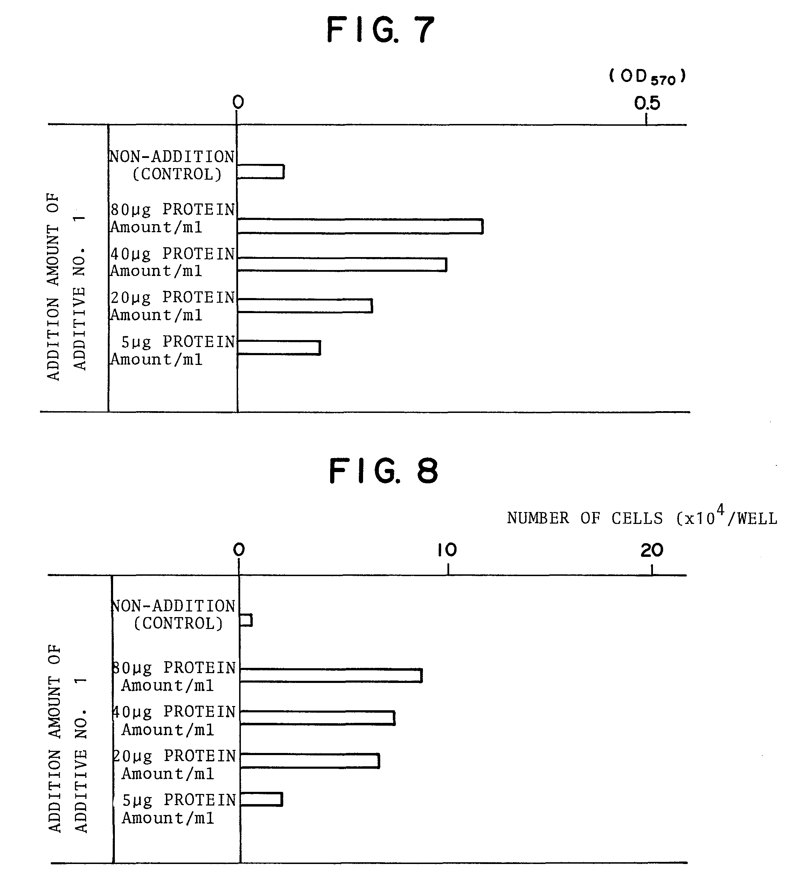

(1)   Each 1 ml of Raji prepared to $1.0 \times 10^5$ cell/ml in PRMI-1640 medium incorporated with "Additive No. 1" at various concentrations was placed on a 24 well plate and cultivated at 37°C in a 5% carbon dioxide gas incubator.  The cell propagating performance 24 hours after the cultivation was determined by MTT Assay.  The results are shown in Figure 7.

(2)   After pipetting each of the wells after 24 hour's cultivation in the test (1), each 100 μl of the cell suspension was taken and mixed with 100 μl of

- 14 -

0.2% tripan blue comprising phosphate buffer solution, which was charged in a Burker-Turk calculator disc and not-dyed number of living cells was counted under microscopic observation. The results are shown in Figure 8.

(3) RPMI-1640 medium in the test (1) was replaced with CEM medium (manufactured by Scoot Co.), which was subjected in the same manner to the test to obtain substantially the same results.

**Test Example 3**

Each 1 ml of highly serum demanding Daudi (Burkitt's human lymphoma cell line: Eur. J. Immunol. 6. (12), 913 - 916 (1976)) prepared to $1.0 \times 10^5$ cell/ml in 10 mM HEPES-comprising RPMI-1640 medium incorporated with "Additive No. 3" at various concentrations was placed on a 24 well plate, cultivated at 37°C in a 5% carbon dioxide gas incubator and the number of cells after three days was measured in the same manner as the Test Example 2-(2). Results are shown in Table 1 below.

Table 1

| "Additive No. 3" addition amount | | Number of cells ($\times 10^5$/ml) |
|---|---|---|
| Non addition (control) | | 0 |
| 50 µg protein amount/ml | | 1.4 |
| 25 | " | 1.2 |
| 12.5 | " | 0.6 |
| 6.3 | " | 0.2 |

**Reference Example 2**

(1)  1000 grams of egg-white were suspended in 481 ml of 10 mM tris-HCl buffer, and polyethylene glycol was added so as to adjust the final concentration to 2.5%, followed by being subjected to centrifugation (10,000 rpm x 10 min) to collect the supernatant. Polyethylene glycol was further added to adjust the concentration to 10% and centrifugation (10,000 rpm x 10 min) was applied to collect the precipitates.  Further, 118 ml of 10 mM tris-HCl buffer added with 1% NaCl were added to dissolve and further subjected in the same manner to centrifugation to collect the supernatant. The supernatant was subjected to dialysis in 10 mM of tris-HCl buffer, then subjected to ion exchange chromatography (DEAE-Trisacryl M, LKB Produkler AB.,
flow rate 75 ml/hr, 50 to 150 mM NaCl gradient, Fr.
10g/tube), fractions No. 6 to 50 were collected,
concentrated and further subjected to gel filtration (CL
6B; flow rate 150ml/hr, Fr. 20g/tube) to obtain 212 mg
of ovomacroglobulin.

(2)  6,541 mg of ovomacroglobulin was obtained by
using 20 Kg of frozen egg-white for foodstuffs (Cupie
Tamago K.K.) and in the same manner as (1) above.

(3)  An equi-volume of 0.16M NaCl aqueous solution
was added to 39 ml of yolk and subjected to
centrifugation (90,000 G x 1 hr, 4°C) to collect an
intermediate layer.  An equi volume of 1.84 M NaCl
aqueous solution was added and further subjected to
centrifugation (90,000 G x 48 hr, 0°C) to collect the
upper layer (oily layer).  After solubilizing the layer
by adding 0.16 M NaCl aqueous solution, dialysis was
applied to the solution, which was sterilized (0.22 μ
millipore filter, Millipore Co.) to obtain 116 ml of
LDL.

Ingredients in LDL (mg/ml)

| | |
|---|---|
| Protein | 20.0 |
| Triglyceride | 49.8 |
| Phospholipid | 16.7 |

| Cholesterol | 3.1 |
| Non esterified fatty acids | 0.23 |

**Example 2**

(1)   Ovomacroglobulin obtained in the Reference Example 2-(1) was subjected to dialysis in 1mM phosphate buffer solution (pH 7.7), then adjusted to 10 mg/ml and, to 20 ml of the resulting solution, added 1 ml of LDL obtained in the Reference Example 2-(3).  This is referred to as "Additive No. 5".

(2)   By using 100 mM HEPES in a physiological saline solution (pH = 7.2), the following composition was prepared:

| Ovomacroglobulin | 2 mg/ml |
| LDL (as the amount of triglyceride) | 500 µg/ml |
| Bovine serum albumin, BSA (Fraction V, #4503:  Sigma Chemical Co.) | 10 mg/ml |
| Catalase | $10^{-6}$ M |
| SGF-3 (Insulin 5 mg/ml, Selenium 5 µg/ml, Transfferin 5mg/ml:  Scoot Co.) | 1% (v/v) |

It is referred to as "Additive No. 6".

(3)   After subjecting ovomacroglobulin obtained in the Reference Example 2-(1) to dialysis in RPMI-1640, it was adjusted to 5.7 mg/ml.  This is referred to as "Additive No. 7".

- 18 -

(4)  A MEM medium comprising ovomacroglobulin of 100 µg/ml as the final content was prepared by using "Additive No. 4".  This is referred to as "Culture medium NO. 1".

(5)  Using RPMI-1640 medium and each of the following ingredients was added so as to adjust the final concentration as:

| | |
|---|---|
| LDL (as the amount of triglyceride) | 50 µg/ml |
| Catalase | $10^{-8}$M |
| Insulin | 5 µg/ml |
| Transfferin | 5 µg/ml |
| Selenium | 5 ng/ml |
| BSA | 1 mg/ml |
| Ovomacroglobulin | 200 µg/ml |

This is referred to as "Culture medium No. 2".

(6)  100 ml of the culture medium was prepared by using RPMI-1640 medium and by adding each of the following ingredients so as to obtain the final concentration:

| | |
|---|---|
| LDL (as the amount of triglyceride) | 250 µg/ml |
| Catalase | $5 \times 10^{-7}$M |
| Insulin | 25 µg/ml |
| Transfferin | 25 µg/ml |
| Selenium | 25 ng/ml |

BSA                                                    5 mg/ml

Ovomacroglobulin                                       1 mg/ml

To 10 ml of the thus prepared culture medium, were added 40 ml of McCoy 5A, 40 ml of L-15 or 40 ml of McCoy 5A + L-15 (1:1) to prepare culture mediums respectively. They are referred to as "Culture medium No. 3", "Culture medium No. 4" and "Culture medium No. 5" respectively.


**Test Example 4**

Daudi subcultured in RPMI-1640 with 10% bovine fetal serum was cultured in "Culture medium No. 2" for 17 days. It was adjusted to have $2 \times 10^{6}$ cell/ml, placed in a 6-well plate by 0.5 ml per well and cultivated in 2.5 ml as final volume of "Culture medium No. 2", "Culture medium No. 3", "Culture medium No. 4" or "Culture medium No. 5" at 37°C in an carbon dioxide gas incubator for 4 days. The number of living cells four days after was determined in the same manner as in the Test Example 2-(2) above. The results are shown in Table 2 below.

0166130

Table 2

| Culture medium | Number of cells $(\times 10^5/\text{ml})$ |
|---|---|
| Culture medium No. 2 | 8.0 |
| Culture medium No. 3 | 9.1 |
| Culture medium No. 4 | 6.2 |
| Culture medium No. 5 | 8.7 |

In the above-mentioned test, additional tests were further carried out by adding known Growth Factor (mitogenic stimulatory factor; MSF, fibroblast growth factor; FGF, epidermal growth factor; EGF, ethanolamine, triiodothyronine; T3 and hydrocortisone) to each of the culture mediums. However, no additional effect was recognized and thus it was found that the culture mediums according to this invention established sufficient culture conditions.

**Test Example 5**

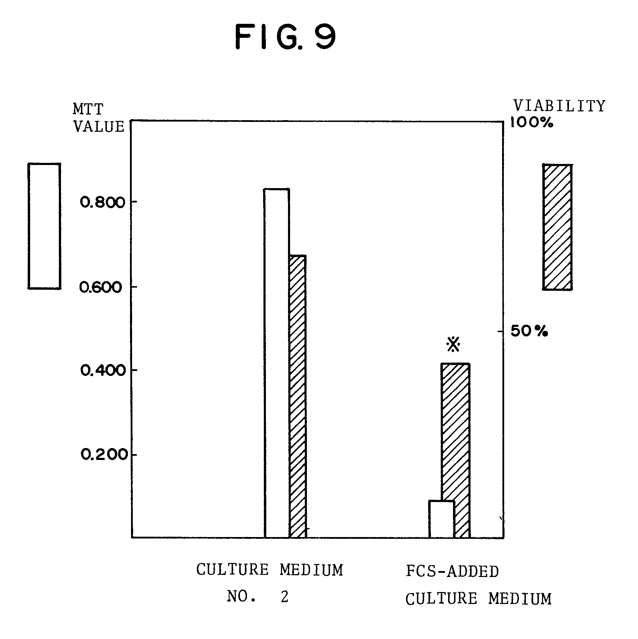

(1) QG-90 adjusted to $1 \times 10^5/\text{ml}$ was cultivated using "Culture medium No. 2" in a 24 well plate. 10 days after the cultivation, nucleo DNA amount was measured by the EB method (JIMRO FBT, Japan Immunoresearch Lab. Co., Ltd.), the number of living cells was determined in the same manner as Test Example 2-(2) and the mitochondria activity (metabolic performance) was measured by the MTT method in the same manner as the Test Example 1-(1) respectively. As for the comparison, test was also carried out using RPMI-1640 with 10% bovine fetal serum. As the result, the nucleo DNA amount by the EB method was substantially the same for both of the culture mediums. The results for the MTT values and the viability (percentage for the number of living cells) are shown in Figure 9. It can be seen from the figure that more preferred culture conditions were established by the culture medium according to this invention as compared with that of the bovine fetal serum added medium. In the determination for the viability in the bovine fetal serum added medium, the cells had no substantial thickness and diminished in the size under microscopic observation and scarcely considered to be living cells. However, since they were not dyed with Trypan blue, they were counted as the living cells.

(2)  The test was carried out quite in the same manner as in (1) above except for using MKN-45

- 22 -

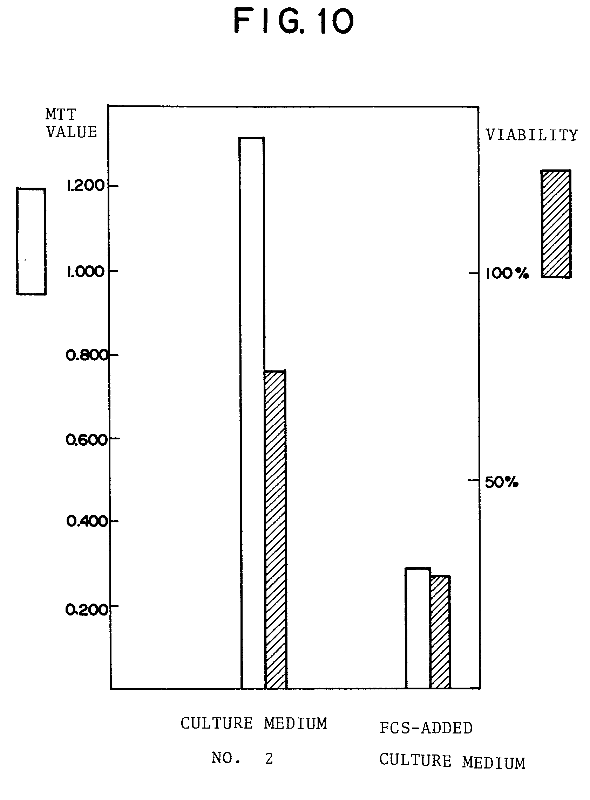

(Tanpakushitsu Kakusan Koso, 23, 6, P697-711 (1978)) adjusted to $0.5 \times 10^5$/ml and measurement was made at 14th day. The results are shown in Figure 10.

**Test Example 6**

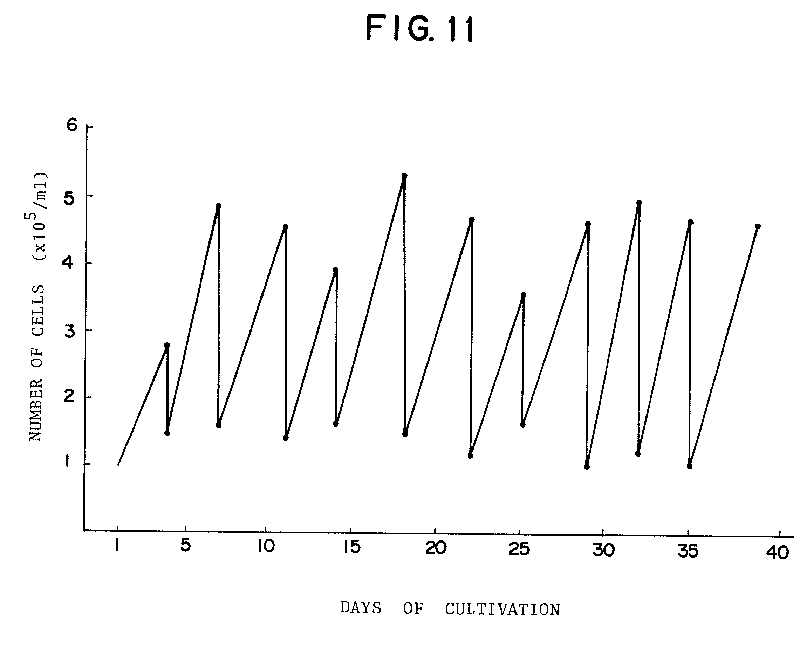

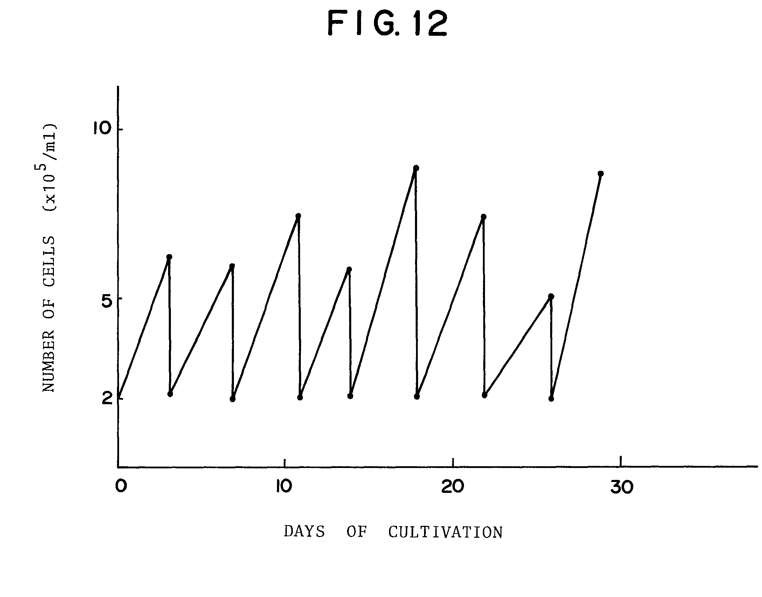

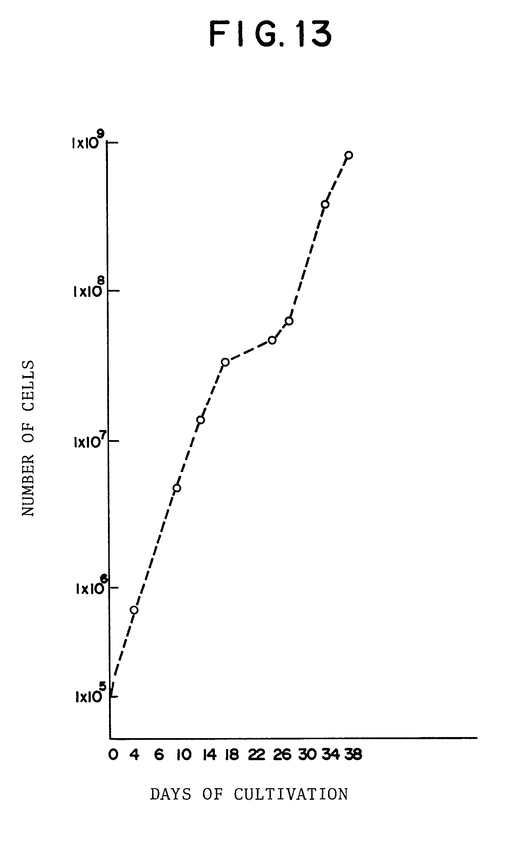

QG-90, Daudi and KATO-III (Jpn. J. Exp. Med., 48, 61-68 (1978)) were subcultivated using "Culture medium No. 2". The results for KATO-III are shown in Figure 11. In the figure, the ordinate indicates the number of living cells determined in the same manner as the Test Example 2-(2) described above. The results for Daudi are shown in Figure 12. The results for QG-90 are shown in Figure 13. In the figures, the ordinate represents the accumulation for the number of living cells.

**WHAT IS CLAIMED IS:**

1.    An additive for use in cell culture comprising ovomacroglobulin.

2.    An additive for use in cell culture comprising an effective amount of ovomacroglobulin.

3.    The additive for use in cell culture as defined in claim 1, further comprising at least one of the ingredients selected from the group consisting of yolk LDL, transferrin, insulin, selenium, albumin and catalase.

4.    The additive for use in cell culture as defined in claim 1, comprising each of the following ingredients each within the range by weight ratio as specified below:

| | |
|---|---|
| Ovomacroglobulin | 1 - 100 |
| Yolk LDL (as the amount of triglyceride) | 0.1 - 500 |
| Catalase | $2.4 \times 10^{-6} - 24$ |
| Alubumin | 10 - 10000 |
| Insulin | 1 - 100 |
| Transferrin | 1 - 100 |
| Selenium | 0.001 - 0.1 |

5. A culture medium for non-serum cultivation containing from 0.1 µg to 1 mg/ml of ovomacroglobulin to the basal medium for cell culture.

6. The culture medium as defined in claim 4, wherein the basal medium for cell culture is a medium selected from the group consisting of CEM, CMRL-1066, DM-160, Eagle's MEN, Autoclavable MEM, Fisher's Medium, F-10, F-12, L-15, NCTC-109, RPMI-1640 and McCoy's 5A.

7. The culture medium as defined in claim 4 or 5 further comprising at least one of the ingredients selected from the group consisting of:

0.1 - 500 µg/ml of yolk LDL as the mount of triglyceride,

1 - 100 µg/ml of transferrin,

1 - 100 g/ml of insulin,

1 - 100 ng/ml of selenium,

10 µg - 10 mg/ml of alubumin and

$1 \times 10^{-14} - 1 \times 10^{-7}$ M of catalase.

8. A method of cell culture under the non-serum condition wherein cells are cultivated in the culture medium as defined in any one of claims 4 through 6.

# FIG. 1

F I G. 2

F I G. 3

FIG. 4

FIG. 5

FIG. 6

# FIG. 7

(OD$_{570}$)

0                                                                    0.5

ADDITION AMOUNT OF ADDITIVE NO. 1

NON-ADDITION (CONTROL)

80μg PROTEIN Amount/ml

40μg PROTEIN Amount/ml

20μg PROTEIN Amount/ml

5μg PROTEIN Amount/ml

# FIG. 8

NUMBER OF CELLS (x10$^4$/WELL

0                          10                         20

ADDITION AMOUNT OF ADDITIVE NO. 1

NON-ADDITION (CONTROL)

80μg PROTEIN Amount/ml

40μg PROTEIN Amount/ml

20μg PROTEIN Amount/ml

5μg PROTEIN Amount/ml

# FIG. 9

7/10

# FIG. 10

# FIG. 11

NUMBER OF CELLS (x10⁵/ml)

DAYS OF CULTIVATION

# FIG. 12

DAYS OF CULTIVATION

0166130

0166130

# FIG.13

NUMBER OF CELLS

$1\times10^9$

$1\times10^8$

$1\times10^7$

$1\times10^6$

$1\times10^5$

0  4  6  10  14  18  22  26  30  34  38

DAYS OF CULTIVATION